# EUROPEAN PATENT APPLICATION

(11) **EP 3 704 941 A1**
(43) Date of publication of application: **09.09.2020**
(21) Application number: 20170970.6
(22) Date of filing: 13.02.2017
(51) Int. Cl.: A01N 25/34, B82B 1/00, B82B 3/00, C23C 22/60, C23C 22/63, B82Y 5/00, B82Y 30/00, B82Y 40/00

(54) **ANTI-BACTERIAL PATTERNED SURFACES AND METHODS OF MAKING THE SAME**

(30) Priority: 12.02.2016 SG 10201601055Y
(62) Divisional of application: 17750533.6
(71) Applicant: Agency for Science, Technology and Research, Singapore 138632 (SG)
(72) Inventor: ZHANG, Yugen, 138669 Singapore (SG); YI, Guangshun, 138669 Singapore (SG)
(74) Representative: Daniels, Stefanie Lisa

(57) **Abstract**

The present invention relates to a substrate comprising a plurality of integrally formed surface features, said surface features being micro-sized and/or nano-sized, said surface features comprising at least one pointed terminus. As a result of this unique surface, said substrate exhibits a biocidal activity because the terminal ends of said surface feature pierce through cell membrane of any microbial cell that comes into contact with the substrate, thereby causing cell deformation and lysis. The present invention also relates to a method producing said substrate. By a simple treatment of copper or zinc foil with a reagent solution comprising an alkali and an oxidizing agent, Cu(OH)₂ nanotube arrays, CuO nano-blades and ZnO nano-needles are prepared. These surfaces are proven to be very effective in killing bacterial (such as *E*. *coli*) *via* a physical interaction.

## Description

### Technical Field

The present invention generally relates to substrates comprising surface features with anti-bacterial properties and methods for preparing the same.

### Background Art

About 80% of infectious diseases caused by microorganisms are spread via contact and thus poses a serious threat to public health. Therefore, killing microorganisms on frequently touched surfaces is an effective way to avoid cross-infection.

A common method to kill microorganisms on such surfaces is by chemical means, such as disinfectants. In another method, antimicrobial surfaces are fabricated by grafting or coating the surfaces with biocidal chemicals or disinfectants to limit cross-infections. However, microorganisms may evolve and develop resistance to the current biocidal chemicals and new chemicals would then need to be developed. Killing via chemical means therefore contributes to secondary contamination. Hence, these methods face challenges such as growing drug resistance to the microbicide agents, low microbial killing efficacy and poor long term stability of coated surfaces.

It was recently discovered that cicada and dragonfly wing surfaces are covered with dense pillared nanostructures that kill microbes or prevent microbial growth by rupturing adhered microbial cells due to a purely physical interaction between the wing surfaces and the microbial cells. The interaction results in cell deformation and lysis without the need for additional external chemical or mechanical means. However, there are presently no known methods that can provide nano-arrayed surfaces capable of physical cell destruction by mimicry of biological surfaces in an efficient and simple manner.

Nanostructures on surfaces of black silicon and TiO₂ have demonstrated microbicidal properties. However, these surface nano-patterns were generated by a top-down approach on specific materials. For example, the black silicon surface was prepared by reactive-ion beam etching on a silicon wafer. Thus, it may be appreciated that the top-down approach would become challenging when nanometer scale patterns are to be generated. In other words, top-down approaches can be time-consuming and expensive and are limited to application on surfaces of specific materials (e.g., those susceptible to etching or other forms of lithographic methods).

Hence, there is a need to provide alternative surfaces demonstrating microbicide properties that overcomes, or at least ameliorates, one or more of the disadvantages described above. There is also a need to provide simple and scalable methods to create such surfaces.

### Summary of Invention

According to a first aspect, there is provided a substrate comprising a plurality of integrally formed surface features, wherein the surface features are micro-sized, nano-sized or a mixture thereof, each surface feature comprising a crystalline phase and at least one pointed terminus.

Advantageously, the surface features are integrally formed, i.e., they form a unitary body with the rest of the substrate. The formation of such surface features does not require the use of stamping techniques to transfer surface features onto the substrate surface.

Advantageously, the terminal ends of said surface features may be adapted to perturb, deform, lyse or damage cell membrane lipid layers to thereby reduce microbe/bacteria viability or cell count. Additionally, the terminal ends may also provide a substrate surface topology that is not conducive for microbes to adhere thereon and which substantially inhibits or prevents microbial cell growth and/or reduces microbe cell count. The interaction between the microbes and surface features may be primarily or exclusively physical in nature. That is, the inhibition or killing of microbes may be achieved via non-chemical means.

Another aspect of the invention relates to a substrate comprising a plurality of integrally formed surface features, wherein the surface features are micro-sized and/or nano-sized, each surface feature comprising a crystalline phase and at least one pointed terminus, and wherein the surface features are formed by, or obtainable from, a one-step process comprising contacting a surface of said substrate with a reagent solution comprising an alkali and an oxidizing agent to thereby integrally form the surface features on the surface of said substrate.

Another aspect relates to a substrate comprising a copper surface, the copper surface comprising a plurality of surface features integrally formed thereon, said surface features being micro-sized and/or nano-sized, wherein said surface features comprises Cu(OH)₂, CuO or a mixture thereof, each Cu(OH)₂ or CuO surface feature comprising at least one pointed terminus.

Still another aspect relates to a substrate comprising a zinc surface, the zinc surface comprising a plurality of micro-sized and/or nano-sized ZnO surface features integrally formed thereon, each ZnO surface feature comprising at least one pointed terminus.

Still another aspect relates to a method of producing a substrate possessing antibacterial properties, the method comprising: contacting a surface of the substrate with a reagent solution to produce a plurality of integrally formed, micro-sized and/or nano-sized surface features on the substrate surface, each surface feature comprising a crystalline phase and at least one pointed terminus.

Yet another aspect relates to a method of producing a substrate possessing antibacterial properties, the method comprising: contacting a surface of the substrate with a reagent solution to produce a plurality of integrally formed, micro-sized or nano-sized surface features by precipitation on the substrate surface, each surface feature comprising a crystalline phase and at least one pointed terminus.

Advantageously, the disclosed methods are capable of providing the surface features having physical dimensions that are adjustable or scalable to exhibit antibacterial properties. For instance, the dimensions may be adjusted by varying the composition/concentration of the reagent solution or the contacting time. Further advantageously, the resolution of these surface features is not limited by the resolution of a mold as is the case when using conventional etching or lithography techniques to form surface features. More advantageously, the disclosed method does not require complex or multi-step nano-imprinting or screen printing methods to obtain nano-sized surface features on the surface of the substrate. Advantageously, the disclosed method can be used with "hard" metal substrates which may not be malleable to conventional surface modification techniques. The disclosed method is also capable of forming these surface features in relative short time periods compared to complex, high resolution lithography techniques (e.g., electron beam lithography). Advantageously, the disclosed method is capable of preparing metal substrates capable of bio-mimicry, e.g., replicating or simulating physical, non-chemical bacteria killing properties found in nature.

The present invention further provides methods of providing antibacterial or antimicrobial properties to a surface by coupling the substrates as disclosed herein to the surface. There is further provided the non-therapeutic use of the substrates disclosed herein for inhibiting the growth of or for killing bacteria or microbes in an ex-vivo environment, e.g., for sterilizing systems, medical kits, equipment, apparel, etc. Alternatively, the substrates as disclosed herein may also be used in therapy, e.g., wound plasters.

### Definitions

The following words and terms used herein shall have the meaning indicated:
The term "microbe" refers to one or a plurality of microorganisms which include bacteria, fungi, algae, yeasts, molds and viruses.
The term "antimicrobial" refers to anything that kills or inhibits the growth of microbes. The term "antimicrobial" can be used to describe a thing or a characteristic of the thing and in this context, refers to the ability to kill or inhibit the growth of microbes. Accordingly, the term "antibacterial" refers to anything that kills or inhibits the growth of bacteria or, when describing a thing or a characteristic of the thing, refers to the ability to kill or inhibit the growth of bacteria. The terms "antimicrobial", "microbicide" and "biocide" are used interchangeably.
The prefix "nano" denotes average sizes of a scale below 1 µm. Accordingly, the term "nano-sized", as used in the context of the specification, refers to a feature having at least one dimension, e.g. length or height, with a nanoscale size. The term "nanostructure" or grammatical variants thereof is to be interpreted accordingly, to refer to a feature or pattern, e.g. blade or tube, having at least one dimension in the nanoscale.
The prefix "micro" denotes average sizes of a scale between about 1 µm to about 1000 µm. Accordingly, the term "micro-sized", as used in the context of the specification, refers to a feature having at least one dimension, e.g. length or height, with a microscale size.
The term "crystalline" or "crystalline phase" as used herein is to be broadly interpreted to refer to a physical state having regularly repeating arrangement of molecules which are maintained over a long range or regularly repeating external face planes. The regularly repeating building blocks are arranged according to well-defined symmetries into unit cells that are repeated.in three-dimensions.
The word "substantially" does not exclude "completely" e.g. a composition which is "substantially free" from Y may be completely free from Y. Where necessary, the word "substantially" may be omitted from the definition of the invention.

Unless specified otherwise, the terms "comprising" and "comprise", and grammatical variants thereof, are intended to represent "open" or "inclusive" language such that they include recited elements but also permit inclusion of additional, unrecited elements.

As used herein, the term "about", in the context of concentrations of components of the formulations, typically means +/- 5% of the stated value, more typically +/- 4% of the stated value, more typically +/- 3% of the stated value, more typically, +/- 2% of the stated value, even more typically +/- 1% of the stated value, and even more typically +/- 0.5% of the stated value.

Throughout this disclosure, certain embodiments may be disclosed in a range format. It should be understood that the description in range format is merely for convenience and brevity and should not be construed as an inflexible limitation on the scope of the disclosed ranges. Accordingly, the description of a range should be considered to have specifically disclosed all the possible sub-ranges as well as individual numerical values within that range. For example, description of a range such as from 1 to 6 should be considered to have specifically disclosed sub-ranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Certain embodiments may also be described broadly and generically herein. Each of the narrower species and sub-generic groupings falling within the generic disclosure also form part of the disclosure. This includes the generic description of the embodiments with a proviso or negative limitation removing any subject matter from the genus, regardless of whether or not the excised material is specifically recited herein.

### Detailed Disclosure of Embodiments

Exemplary, non-limiting embodiments of a substrate comprising a plurality of integrally formed surface features will now be disclosed.

In embodiments, there is provided a substrate comprising a plurality of integrally formed surface features, said surface features being micro-sized and/or nano-sized, each surface feature comprising a crystalline phase and at least one pointed terminus.

The disclosed substrate may be made from a large variety of materials. For example, the substrate may comprise a metal or a polymer. In another example, the substrate may comprise at least one metal, one polymer, mixtures of metals, mixtures of polymers or mixtures of polymers and metals.

In some embodiments, the disclosed substrate may be capable of supporting the growth of the plurality of surface features on its surface. In an embodiment, the substrate may be capable of supporting the growth of salts of the substrate on its surface. Advantageously, the surface structures may be integrally formed by precipitation of the salts on the substrate. Therefore, any substrate capable of supporting the deposition of surface structures or features comprising salts may be suited for the present disclosure.

In other embodiments, the disclosed substrate may be capable of reaction to thereby integrally form the plurality of surface features on its surface. In an embodiment, the surface of the substrate may be reactive with an oxidizing agent to thereby integrally form the plurality of surface features. Advantageously, the surface structures may be integrally formed by straightforward reaction of the substrate with an oxidizing agent to obtain salts of the substrate. Therefore, any substrate capable of forming surface structures or features when oxidized may be suited for the present disclosure.

In an embodiment, the substrate comprises a metal surface comprising any suitable reactive metal capable of forming an insoluble salt with an oxidizing agent. In another embodiment, the substrate comprises a metal surface comprising any suitable metal capable of supporting the growth of an insoluble salt thereon. In an example, the substrate comprising a metal surface may comprise a divalent metal. In another example, the substrate comprising a metal surface may comprise a transition metal selected from Group 11 of the periodic table, such as Cu, or Group 12 of the periodic table, such as Zn.

In embodiments, the metal may be an alloy or a multi-layered structure, optionally comprising at least one oxidizable metal surface. The metal may include aluminum-based alloys, copper-based alloys, iron-based alloys, nickel-based alloys, titanium-based alloys, tin-based alloys, zinc-based alloys, steel, brass or hastelloy. The metal may include two or more metals selected from the group consisting of transition metals, rare earth metals, aluminium, copper, iron, nickel, titanium, tin, zinc, manganese, chromium, carbon, silicon, tungsten and other suitable alloy metals.

The substrate surface may be coated with one or more layers of reactive or oxidative solution to thereby integrally form the plurality of surface features on its surface. The oxidation of the substrate surface may form salts or salt crystals which are insoluble in typical organic or inorganic solvents or aqueous mediums that contact the surfaces. Hence in an embodiment, the crystalline phase of the surface feature may comprise the insoluble salt formed from the oxidation of the surface. In some embodiments, the substrate surface may be coated with one or more layers of reagent solution comprising ions of salts which are insoluble in typical organic or inorganic solvents or aqueous mediums that contact the surfaces. Hence in an embodiment, the crystalline phase of the surface feature may comprise the insoluble salt formed by precipitation or deposition onto the substrate surface. For example, the salt or salt crystals may be insoluble in rain water, fruit juices or perspiration. Therefore, the disclosed substrate may advantageously be weather resistant and the antimicrobial and antibacterial properties of the disclosed substrate may be long-lasting. The formed surface features are advantageously ordered and crystalline, which otherwise would be difficult to obtain with top-down surface modification techniques.

In an embodiment, the crystalline phase of the surface feature may comprise an oxide salt or a hydroxide salt. Advantageously, the oxide and hydroxide surface features may be formed *in-situ* via oxidation reactions, acid/base reactions or salt precipitation reactions. Advantageously, the fabrication of such surface features does not require complex techniques, e.g., plasma etching, reactive ion etching, physical or chemical vapor deposition techniques or lithography techniques. In one embodiment, the oxide and/or hydroxide features may be formed via a one-pot or one-step reaction synthesis. The oxide or hydroxide surface features may advantageously be formed of a simple oxidation or precipitation reaction.

Accordingly in an embodiment, there is provided a substrate comprising a plurality of integrally formed surface features, wherein the surface features are micro-sized and/or nano-sized, each surface feature comprising a crystalline phase and at least one pointed terminus, and wherein the surface features are formed by, or obtainable from, a one-step process comprising contacting a surface of said substrate with an oxidizing solution comprising an alkali and an oxidizing agent to thereby integrally form the surface features on the surface of said substrate. It is postulated that the process of contacting the surface of the substrate with an oxidizing solution comprising an alkali and an oxidizing agent results in the formation of the plurality of surface features, each comprising at least one pointed terminus. Due to the nature of their chemical formation, the exact characterization of the structure of each surface feature formed from the process may not be exhaustively described by physical characteristics, although exemplary and optional embodiments of the surface features are described below.

Accordingly in another embodiment, there is provided a substrate comprising a plurality of integrally formed surface features, wherein the surface features are micro-sized and/or nano-sized, each surface feature comprising a crystalline phase and at least one pointed terminus, and wherein the surface features are formed by, or obtainable from, a one-step process comprising contacting a surface of said substrate with a reagent solution comprising ions of salts to thereby integrally form the surface features by precipitation on the surface of said substrate. It is postulated that the process of contacting the surface of the substrate with a reagent solution comprising ions of salts results in the formation of the plurality of surface features, each comprising at least one pointed terminus, deposited or precipitated on the substrate surface. Due to the nature of their chemical formation, the exact characterization of the structure of each surface feature formed from the process may not be exhaustively described by physical characteristics, although exemplary and optional embodiments of the surface features are described below.

Each surface feature comprises at least one pointed terminus. The terminus or distal end of the integrally formed surface feature is an end opposite the substrate, facing away from the substrate. Upon physical contact with microbial cells, the pointed terminus or protrusion is advantageously effective in rupturing the cell walls and thereby killing or at least inhibiting the growth of the cells. Accordingly, any microbe transferred to or contacting the disclosed substrate may advantageously be killed or inhibited from growing. Thus, the spread of infectious diseases caused by microbes may advantageously be stopped or at least slowed down.

The surface feature may comprise a crystalline phase that provides the at least one pointed terminus. The crystalline phase may be selected from an orthorhombic crystal structure, monoclinic crystal structure, triclinic crystal structure, tetragonal crystal structure, hexagonal crystal structure, trigonal crystal structure or cubic crystal structure. In an embodiment, the crystalline phase has a structure selected from an orthorhombic crystal structure, monoclinic crystal structure or a hexagonal crystal structure. An example of a hexagonal crystal system is a wurtzite crystal structure. An example of an orthorhombic crystal structure is one having an X-Ray Diffraction characterization of JCPDS no. 13-0420. An example of a monoclinic crystal structure is one having an X-Ray Diffraction characterization of JCPDS no. 48-1548.

The surface feature may be of a shape that provides the at least one pointed terminus. The integrally formed surface feature may be tapered in shape, having a base end coupled to a surface of the substrate and a distal end that is smaller in dimension relative to the base end. For example, the surface feature may have a shape selected from the group consisting of tubes, blades, needles, pyramids, cones, pillars and mixtures thereof. Hence, the distal end of the surface feature may refer to a tapered tip, a bladed end, a conical apex, or a pyramidal vertex. Preferably, the distal end refers to a pointed terminus of a surface feature.

In an embodiment, the surface feature is a nanotube or a needle. The nanotube or needle may be tapered or may comprise a distal end having a smaller cross-sectional diameter compared to a cross-sectional diameter of its base section. The corresponding distal end may be of circular cross-section having a diameter. In another embodiment, the surface feature is a blade and the corresponding distal end may be of a rectangular cross-section having a breadth or thickness.

Exemplary dimensions of the surface features may be provided as follows.

The dimensions of the surface feature may be in the micro-size scale or in the nano-size scale or a mixture of micro-size and nano-size scales. The dimensions of the surface features may be advantageously tailored according to, for example, the application of the substrate or the size of the microbe(s) intended for killing or inhibition.

The ratio of the height of the surface feature to a dimension of the terminus distal end of the surface feature may be about 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190 or 200. The ratio of the height of the surface feature to a dimension (e.g. diameter or thickness) of the terminus distal end of the surface feature may be in a range comprising an upper and lower limit selected from any two of the above values.

The height or length of the surface feature refers to a dimension from the base of the surface feature formed at the substrate surface to the distal end or terminus of the surface feature. In the context of the present disclosure, the higher the ratio of surface feature height to a dimension of the distal end of the surface feature, the sharper would be the distal end of the surface feature. A higher ratio of surface feature height to a dimension of the distal end of the surface feature signifies a higher sharpness of the distal end of the surface feature. Advantageously, it has been discovered that the sharpness of the distal end of the surface feature is proportional to the antimicrobial efficiency of the substrate. That is, the sharper the pointed terminus, the more effective the surface feature would be in killing or inhibiting the growth of the cells. Advantageously, the disclosed substrate is capable of reducing an amount of bacteria contacting the substrate to 0.5 or less of the initial CFU value per unit volume. The surface features of the disclosed substrate may have a sharpness higher than known natural or artificial biocidal surfaces. Thus, the antimicrobial efficiency of the disclosed substrate may be higher than the known biocidal surfaces.

The surface feature may possess a height selected from about 200 nm, 300 nm, 400 nm, 500 nm, 600 nm, 700 nm, 800 nm, 900 nm, 1 µm, 1.25 µm, 1.5 µm, 1.75 µm, 2 µm, 2.25 µm, 2.5 µm, 2.75 µm, 3 µm, 3.25 µm, 3.5 µm, 3.75 µm, 4 µm, 4.25 µm, 4.5 µm, 4.75 µm, 5 µm, 5.25 µm, 5.5 µm, 5.75 µm, 6 µm, 6.25 µm, 6.5 µm, 6.75 µm, 7 µm, 7.25 µm, 7.5 µm, 7.75 µm, 8 µm, 8.25 µm, 8.5 µm, 8.75 µm, 9 µm, 9.25 µm, 9.5 µm, 9.75 µm or 10 µm. The surface feature may possess a height in a range comprising an upper limit and a lower limit selected from any two of the above values.

In embodiments, the dimension of the distal end may refer to a cross-sectional diameter, a width, or a thickness of the distal end. In an embodiment, the dimension of the distal end of the surface feature is selected from diameter or thickness. The dimension of the terminus distal end of the surface feature, that is in an embodiment the diameter or thickness of the terminus distal end, is selected from about 1 nm to about 500 nm, or about 5 nm, 10 nm, 15 nm, 20 nm, 25 nm, 30 nm, 35 nm, 40 nm, 45 nm, 50 nm, 55 nm, 60 nm, 65 nm, 70 nm, 75 nm, 80 nm, 85 nm, 90 nm, 95 nm, 100 nm, 105 nm, 110 nm, 115 nm, 120 nm, 125 nm, 130 nm, 135 nm, 140 nm, 145 nm, 150 nm, 155 nm, 160 nm, 165 nm, 170 nm, 175 nm, 180 nm, 185 nm, 190 nm, 195 nm, 200 nm, 205 nm, 210 nm, 215 nm, 220 nm, 225 nm, 230 nm, 235 nm, 240 nm, 245 nm, 250 nm, 255 nm, 260 nm, 265 nm, 270 nm, 275 nm, 280 nm, 285 nm, 290 nm, 295 nm, 300 nm, 305 nm, 310 nm, 315 nm, 320 nm, 325 nm, 330 nm, 335 nm, 340 nm, 345 nm, 350 nm, 355 nm, 360 nm, 365 nm, 370 nm, 375 nm, 380 nm, 385 nm, 390 nm, 395 nm, 400 nm, 405 nm, 410 nm, 415 nm, 420 nm, 425 nm, 430 nm, 435 nm, 440 nm, 445 nm, 450 nm, 455 nm, 460 nm, 465 nm, 470 nm, 475 nm, 480 nm, 485 nm, 490 nm, 495 nm or 500 nm. The dimension of the terminus distal end of the surface feature may be in a range comprising an upper limit and a lower limit selected from any two of the above values. The dimension of the distal end may advantageously be nano-sized. Advantageously, it has been shown that substrates with surface features having tapered ends of between about 10 nm and 400 nm, about 10 nm and 300 nm or about 10 nm and 200 nm in dimension are capable of killing between 90 - 100% of the bacteria S. *aureus* after just one hour of incubation. Further advantageously, the resolution (and size) of the surface features of the present disclosure are not limited by the resolution provided by conventional surface modification techniques. Even further advantageously, the *in-situ* formation of surface features via chemical reaction allows the formation of surface features having terminal dimensions as small as 10 nm.

In an example, when the surface feature is a tube, the height of the tube may range from about 1 µm to 10 µm or about 5 µm to 7 µm; and the distal end may be a tip of circular cross-section having a diameter of from about 50 nm to 300 nm or about 100 nm to 200 nm.

In another example, when the surface feature is a blade, the blade may have a length of from about 200 nm to 5 µm or about 400 nm to 1 µm; and a breadth of from about 100 nm to 500 nm or about 200 nm to 400 nm. The thickness of the blade may be tapered towards the distal end of the blade. The distal end of the blade may be a bladed end having a thickness of from about 10 nm to 30 nm, or about 20 nm.

In yet another example, when the surface feature is a needle, the length of the needle may range from about 500 nm to 5 µm or about 1 µm to 2 µm; the distal end may be a tip of circular cross-section having a diameter of about 1 nm to 100 nm or about 10 nm to 40 nm; and the base or root of the needle may be of circular cross-section having a diameter of about 10 nm to 500 nm or about 100 nm to 200 nm.

The pitch of adjacent surface features may be selected from about 100 nm, 200 nm, 300 nm, 400 nm, 500 nm, 600 nm, 700 nm, 800 nm, 900 nm, 1000 nm, 1100 nm, 1200 nm, 1300 nm, 1400 nm, 1500 nm, 1600 nm, 1700 nm, 1800 nm, 1900 nm or 2000 nm. The pitch of adjacent surface features may be in a range comprising an upper limit and a lower limit selected from any two of the above values.

As microbial and bacterial cells are typically larger than the disclosed pitch, surface features with the disclosed pitch are advantageously capable of contacting and rupturing the cells, thereby conferring antimicrobial and antibacterial properties on the substrate.

In an embodiment, there is provided a substrate comprising a copper surface, the copper surface comprising a plurality of surface features integrally formed thereon, the surface features being micro-sized and/or nano-sized, and wherein the surface features comprise Cu(OH)₂, CuO or a mixture thereof, each Cu(OH)₂ or CuO surface feature comprising at least one pointed terminus. In another embodiment, there is provided a substrate comprising a zinc surface, said zinc surface comprising a plurality of micro-sized and/or nano-sized ZnO surface features integrally formed thereon, said ZnO surface features comprising at least one pointed terminus.

Advantageously, copper and zinc are surface materials commonly encountered in daily life, e.g. doors and doorknobs comprise Cu surfaces, street lamp poles and highway guardrails comprise galvanized steel with Zn surfaces. Hence, it is an advantage that the present disclosure can be applied to common surfaces, such as copper and zinc surfaces, to provide microbicidal surfaces effective in killing or at least inhibiting the growth of microbes via physical means or physical interaction.

Zinc or copper substrates have been advantageously found to provide ease of fabricating the disclosed surface features using straightforward synthesis steps. The use of zinc or copper substrates avoids the need for top-down texturing techniques, e.g., reactive-ion beam etching commonly employed on silicon based substrates. The resolution and size of the surface features of the present disclosure are also advantageously not limited by the resolution provided by conventional surface modification techniques.

Exemplary, non-limiting embodiments of a method of producing a substrate possessing antimicrobial or antibacterial properties will now be disclosed.

In embodiments, there is provided a method of producing a substrate possessing antimicrobial or antibacterial properties, the method comprising: contacting a surface of the substrate with a reagent solution to produce a plurality of integrally formed, micro-sized or nano-sized surface features on the substrate surface, each surface feature comprising a crystalline phase and at least one pointed terminus.

In embodiments, there is provided a method of producing a substrate possessing antimicrobial or antibacterial properties, the method comprising: contacting a surface of the substrate with a reagent solution to produce a plurality of integrally formed, micro-sized or nano-sized surface features by precipitation on the substrate surface, each surface feature comprising a crystalline phase and at least one pointed terminus.

Exemplary reactions at the surface of a copper or zinc substrate are shown below:

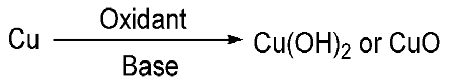

Zn²⁺ + 4OH⁻ → Zn(OH)₄²⁻ + NO₃⁻ → ZnO

The reagent solution may comprise an oxidizing agent selected from halogens, oxygen, peroxides, hypohalites, chlorates, chromates, persulfates, permanganates, nitrates or nitric acid. Examples include ammonium persulfate, zinc nitrate, hydrogen peroxide and sodium hypochlorite.

The concentration of the oxidizing agent in the reagent solution may be selected from about 0.01 M to about 10 M, or 0.02 M, 0.04 M, 0.06 M, 0.08 M, 0.1 M, 0.12 M, 0.14 M, 0.15 M, 0.16 M, 0.17 M, 0.18 M, 0.19 M, 0.2 M, 0.3 M, 0.4 M, 0.5 M, 1.0 M, 1.5 M, 2.0 M, 2.5 M, 3.0 M, 3.5 M, 4.0 M, 4.5 M, or 5.0 M. The concentration of the oxidizing agent in the reagent solution may be in a range comprising an upper limit and a lower limit selected from any two of the above values. Advantageously, the concentration of the oxidizing agent may be suitably selected to provide specific surface feature dimensions as required by the application of the produced metal substrate. In embodiments, a higher concentration of the oxidizing agent may be selected to result in surface features comprising a monoclinic crystal structure, while a lower concentration of the oxidizing agent may be selected to result in surface features comprising an orthorhombic crystal structure. For example, where the concentration of the oxidizing agent is at least about 0.3 M, surface features comprising a monoclinic crystal structure may be obtained.

The reagent solution may comprise a base or alkali. The base may be a strong base having a pK_{b} value of 10 or more. The base may be selected from a base of an alkali metal or of an alkaline earth metal. Examples include NaOH and KOH.

The concentration of the alkali in the reagent solution may be selected from about 1.0 M to about 10 M, or 1.5 M, 2.0 M, 2.5 M, 3.0 M, 3.5 M, 4.0 M, 4.5 M, 5.0 M, 5.5 M, 6.0 M, 6.5 M, 7.0 M, 7.5 M, 8.0 M, 8.5 M, 9.0 M, 9.5 M or 10 M. The concentration of the alkali in the reagent solution may be in a range comprising an upper limit and a lower limit selected from any two of the above values. Advantageously, the concentration of the alkali may be suitably selected to provide specific surface feature dimensions as required by the application of the produced metal substrate. In embodiments, a higher concentration of the alkali may be selected to result in surface features comprising a monoclinic crystal structure, while a lower concentration of the alkali may be selected to result in surface features comprising an orthorhombic crystal structure. For example, where the concentration of the alkali in the reagent solution is in a range of from about 5.0 M to about 10 M, or at least about 5.5 M, 6.0 M, 6.5 M, 7.0 M, 7.5 M, 8.0 M, 8.5 M, 9.0 M, 9.5 M or at least 10 M, surface features comprising a monoclinic crystal structure may be obtained.

In embodiments where the reagent solution comprises both an oxidizing agent and a base, the mole ratio of the oxidizing agent to the base may range from about 1:10 to 1:30, or about 1:12, 1:14, 1:1, 1:18, 1:20, 1:22, 1:24, 1:26, 1:28 or 1:30, or may be in a range comprising an upper limit and a lower limit selected from any two of the above values.

The reagent solution may further comprise water. The concentration of the reagent solution may be adjusted by addition of water.

In other embodiments, the reagent solution may comprise other reagents to provide ions of salts, such as cations and anions that form insoluble salts. Suitable cations may be metal ions of the metals disclosed herein. Suitable anions may be nitrate ions, hydroxide ions or carbonate ions.

The concentration of the cation source in the reagent solution may be selected from about 0.01 M to about 5 M, or 0.02 M, 0.04 M, 0.06 M, 0.08 M, 0.1 M, 0.12 M, 0.14 M, 0.15 M, 0.16 M, 0.17 M, 0.18 M, 0.19 M, 0.2 M, 0.3 M, 0.4 M, 0.5 M, 1.0 M, 1.5 M, 2.0 M, 2.5 M, 3.0 M, 3.5 M, 4.0 M, 4.5 M, or 5.0 M. The concentration of the cation source in the reagent solution may be in a range comprising an upper limit and a lower limit selected from any two of the above values. Advantageously, the concentration of the oxidizing agent may be suitably selected to provide specific surface feature dimensions as required by the application of the produced metal substrate. In an example, the concentration of zinc nitrate as a cation source may be selected from about 0.01 M to about 5 M inclusive, or any concentration in between.

In some embodiments, the reagent solution may not comprise an oxidizing agent but may comprise ions of insoluble salts capable of precipitating on the substrate surface. In some embodiments, the reagent solution may not comprise an oxidizing agent but may comprise ions of insoluble salts capable of precipitating on the substrate surface and a base.

In a particular embodiment, the reagent solution may comprise zinc nitrate and a base as disclosed herein, such as KOH, wherein the zinc ion and the hydroxide ion ultimately results in the insoluble zinc oxide salt precipitated on the substrate surface. In this embodiment, the reagent solution may not comprise an oxidizing agent.

The contacting step may be conducted for a duration sufficient to produce the plurality of surface features. The duration may be suitably selected to provide specific surface feature dimensions as required by the application of the produced metal substrate. The duration may be suitably selected depending on the substrate material. The contacting step may be conducted for a duration of about 10 minutes, 20 minutes, 30 minutes, 40 minutes, 50 minutes, 60 minutes, 70 minutes, 80 minutes, 90 minutes, 100 minutes, 110 minutes, 120 minutes, 130 minutes, 140 minutes, 150 minutes, 160 minutes, 170 minutes, 180 minutes, 190 minutes, 200 minutes, 210 minutes, 220 minutes, 230 minutes, 240 minutes, 250 minutes, 260 minutes, 270 minutes, 280 minutes, 290 minutes, 300 minutes, 310 minutes, 320 minutes, 330 minutes, 340 minutes, 350 minutes, 360 minutes, 370 minutes, 380 minutes, 390 minutes, 400 minutes, 410 minutes, 420 minutes, 430 minutes, 440 minutes, 450 minutes, 460 minutes, 470 minutes, 480 minutes, 540 minutes, 600 minutes, 660 minutes, 720 minutes, 780 minutes, 840 minutes, 900 minutes, 960 minutes, 1020 minutes, 1080 minutes, 1140 minutes, 1200 minutes, 1260 minutes, 1320 minutes, 1380 minutes or 1440 minutes. The contacting step may be conducted for a duration in a range comprising an upper limit and a lower limit selected from any two of the above values. In an example, where the substrate is copper and surface features comprising an orthorhombic crystal structure are desired, the contacting step may be conducted for a duration of about 10 to 20 minutes. In another example, where the substrate is copper and surface features comprising a monoclinic crystal structure are desired, the contacting step may be conducted for a duration of about 25 to 35 minutes. In an example, where the substrate is zinc and surface features comprising a wurtzite crystal structure are desired, the contacting step may be conducted for a duration of about 6 to 18 hours.

Accordingly, the concentration of the alkali, or the concentration of the oxidizing agent, or the concentration of the ions, or the duration of the contacting step, or the temperature of the contacting step, or any combination thereof, may be selected to provide specific surface feature dimensions as required by the application of the produced metal substrate. In embodiments, an increase in the concentration of the alkali, or an increase in the concentration of the concentration of the oxidizing agent, or an increase in both the concentrations of the alkali and the oxidizing agent may result in surface features comprising a monoclinic crystal structure.

The contacting step may be conducted at room temperature or ambient temperature, or about 15°C, or about 20°C, or about 25°C, or about 30°C. Advantageously, the disclosed method is capable of being conducted without the use of specialized equipment, such as pressurized chambers or heat-rated vessels.

The substrate may be transformed into a substrate possessing antimicrobial/antibacterial properties using the disclosed one-step method. In one embodiment, the surface features may be formed via a one-pot or one-step reaction synthesis. Thus, the disclosed surface features may be formed *in-situ* via simple oxidation reactions or acid/base reactions or precipitation reactions. The disclosed method is therefore advantageous and cost-effective over known complex techniques of fabricating surface features on metal substrates. Advantageously, the disclosed method is capable of providing the surface features without being limited by the resolution of a mold as required by conventional etching or lithography techniques. Advantageously, the disclosed method does not require complex or multi-step nano-imprinting or screen printing methods to obtain nano-sized surface features on the surface of the substrate. Advantageously, fabrication of the disclosed surface features does not require complex techniques, e.g., plasma etching, reactive ion etching, physical or chemical vapor deposition techniques. Advantageously, the disclosed method can be used with "hard" metal substrates that may not be malleable to conventional surface modification techniques. Advantageously, the disclosed method is capable of preparing metal substrates capable of bio-mimicry, e.g., replicating or simulating physical, non-chemical microbe/bacteria-killing properties found in nature.

The substrate may be one as disclosed herein. For example, the substrate may comprise a metal surface, such as a transition metal surface, said surface optionally being oxidisable to form insoluble salts to integrally form the surface features thereon. Examples of transition metal surfaces include transition metals selected from Group 11 of the periodic table, e.g. Cu, or Group 12 of the periodic table, e.g. Zn.

The surface feature may be one as disclosed herein. For example, where the substrate comprises a metal surface, the surface feature may comprise oxide and/or hydroxide salts of the metal.

The present disclosure further provides a substrate comprising a metal surface, the metal surface comprising a plurality of integrally formed, micro-sized and/or nano-sized surface features, said substrate being obtainable by a method as disclosed herein.

The present disclosure provides the use of a substrate as disclosed herein for providing antimicrobial and antibacterial properties to an ex-vivo environment. The disclosed substrate may provide bacteriostatic or bactericidal purposes to the ex-vivo environment. Accordingly, as the use of the substrate is in an ex-vivo environment, the use may be a non-therapeutic one.

Alternatively, the disclosed substrate may be used in therapy. The disclosed substrate may be used in the treatment of microbial infections.

The disclosed substrate may be capable of killing or inhibiting the growth of microbes. The microbes may be pathogenic or non-pathogenic. The microbes may be bacteria or fungi. The bacteria may include gram-negative and gram-positive bacteria.

Examples of gram-positive bacteria include Staphylococcus, Enterococcus and Streptococcus, such as *Staphylococcus aureus, Enterococcus faecalis, Bacillus megaterium, Hay bacillus, Mycobacterium smegmatis* and *Streptococcus pneumoniae.* Examples of gram-negative bacteria include Escherichia, Shigella and Salmonella, such as *Escherichia coli, Pseudomonas aeruginosa, Chlamydia trachomatis, Helicobacter pylori, Shigella dysenteriae, Salmonella enteritidis* and *Salmonella typhi.*

### Brief Description of Drawings

The accompanying drawings illustrate a disclosed embodiment and serves to explain the principles of the disclosed embodiment. It is to be understood, however, that the drawings are designed for purposes of illustration only, and not as a definition of the limits of the invention.
**Fig. 1**

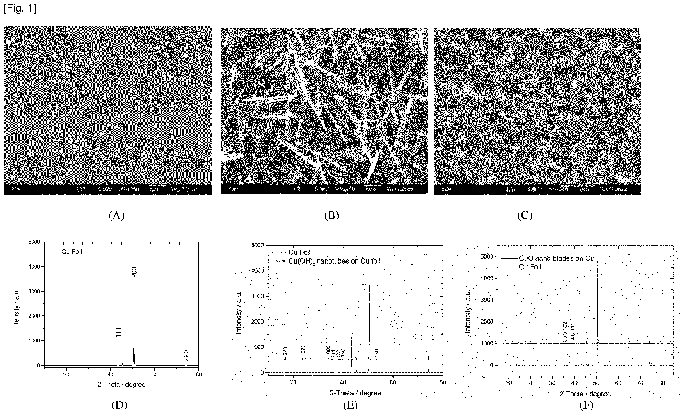

   [Fig. 1] contains the Scanning Electron Microscopy (SEM) images of (A) Cu foil, (B) Cu(OH)₂ nanotubes growing on Cu foil, (C) CuO nano-blades growing on Cu foil and the graphs of their corresponding X-Ray Diffraction (XRD) patterns (D-F), confirming their respective structures.
**Fig. 2**

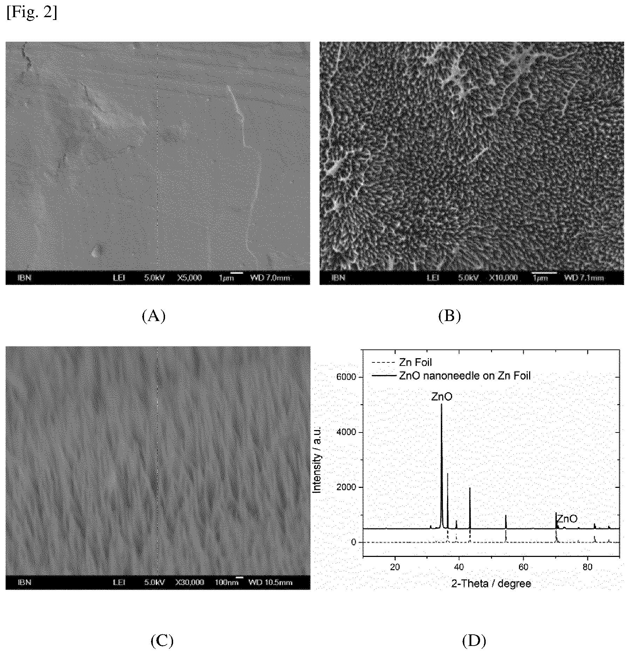

   [Fig. 2] contains the SEM images of (A) Zn foil, (B, C) ZnO nano-needles growing on Zn foil, and (D) graph of the XRD pattern of ZnO nano-needles on Zn foil.
**Fig. 3**

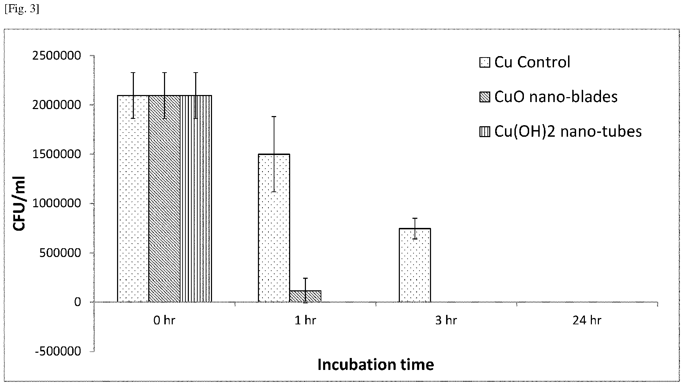

   [Fig. 3] is a graph of the Colony Forming Units (CFU)/ml against the incubation time showing the killing efficacy (against E. *coli*) of various copper surfaces evaluated using Japanese Industrial Standard (JIS) Z 2801/ISO 22196 method.
**Fig. 4**

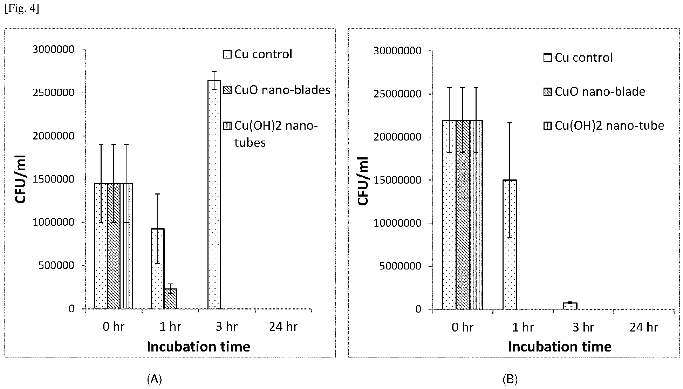

   [Fig. 4] contains graphs of the CFU/ml against the incubation time demonstrating the killing efficacy (against *E*. *coli*) of various copper surfaces evaluated using JIS Z 2801/ISO 22196 method for (A) samples with Pt coating and (B) samples with Cu coating.
**Fig. 5**

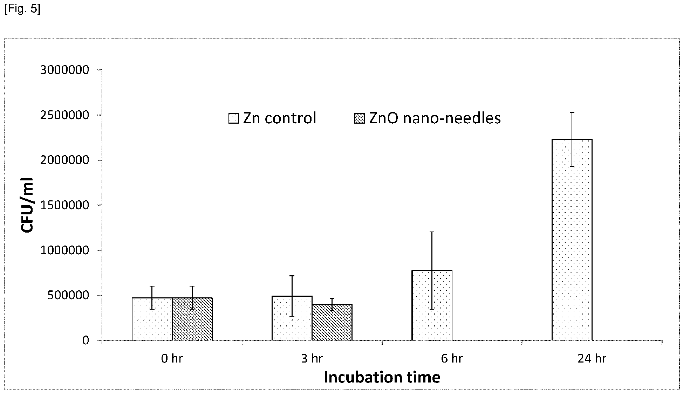

   [Fig. 5] is a graph of the CFU/ml against the incubation time showing the killing efficacy (against *E*. *coli*) of flat Zn foil and ZnO nano-needle surface evaluated using JIS Z 2801/ISO 22196 method.
**Fig. 6**

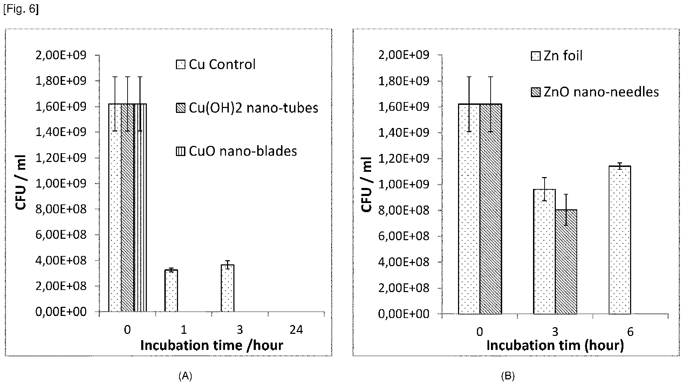

   [Fig. 6] contains graphs of the CFU/ml against the incubation time demonstrating the killing efficacy (against *S*. *aureus*) of (A) flat Cu foil, Cu(OH)₂ nano-tubes, CuO nano-blades surface, and (B) flat Zn foil and ZnO nano-needle surface evaluated using JIS Z 2801/ISO 22196 method.
**Fig. 7**

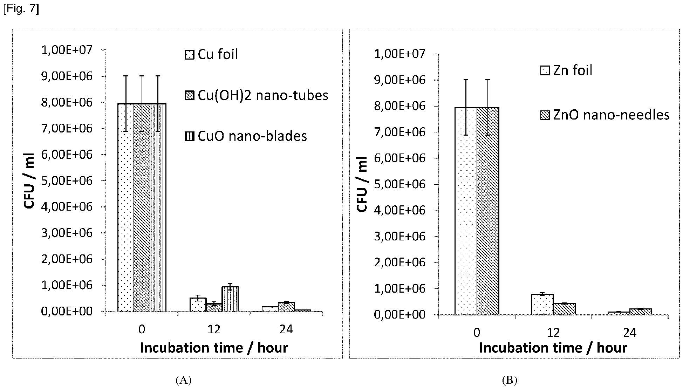

   [Fig. 7] contains graphs of the CFU/ml against the incubation time demonstrating the killing efficacy (against *C. albicans*) of (A) flat Cu foil, Cu(OH)₂ nano-tubes, CuO nano-blades surface, and (B) flat Zn foil and ZnO nano-needle surface evaluated using JIS Z 2801/ISO 22196 method.
**Fig. 8**

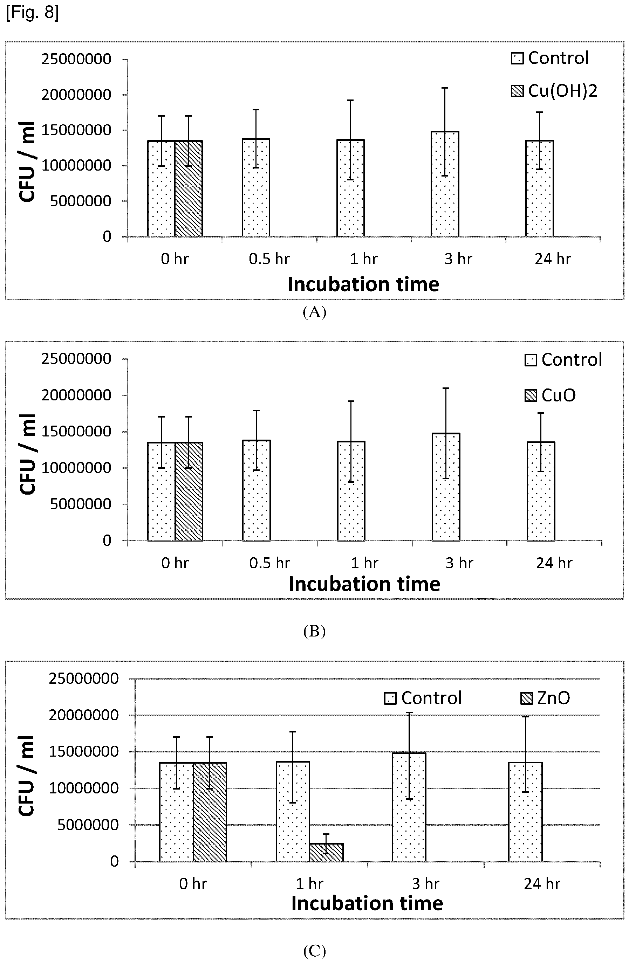

   [Fig. 8] contains graphs of the CFU/ml against the incubation time demonstrating the killing profiles (against *E*. *coli*) of nano-structured surfaces (A) Cu(OH)₂ nanotubes surface, (B) CuO nano-blades surface, and (C) ZnO nano-needles surface in water under shaking condition. Testing conditions: 5 ml water, 37 °C, shaking at 300 r/min.

### Examples

Non-limiting examples of the invention and a comparative example will be further described in greater detail by reference to specific Examples, which should not be construed as in any way limiting the scope of the invention.

### Example 1: Preparation of Cu(OH)₂ nanotubes and CuO nanoblades on Cu substrate

For the growing of Cu(OH)₂ nanotubes, 4 ml of 1M (NH₄)₂S₂O₈, 8 ml of 10M NaOH and 18 ml of water were mixed to form a solution. A Cu foil (20 x 25 mm) was suspended in the solution for 15 min. A solid film of Cu(OH)₂ nanotubes was obtained on the Cu foil. The Cu foil was then washed 3 times with water and 3 times with ethanol. After washing, the foil was dried with flowing N₂ and stored for future use.

For the growing of CuO nanoblades, 4 ml 1M (NH₄)₂S₂O₈ solution and 8 ml 10M NaOH were mixed. A Cu foil (20 x 25 mm) was suspended in the solution for 30 min. A black solid film of CuO nanoblades was obtained on the Cu foil. The Cu foil was then washed 3 times with water and 3 times with ethanol. After washing, the foil was dried with flowing N₂ and stored for future use.

### Example 2: Preparation of ZnO nanoneedles on Zn substrate

For the growing of ZnO nanoneedles, 10 ml of 0.5M Zn(NO₃)₂ aqueous solution and 10 ml 4M KOH were mixed. A Zn foil (20 x 20 mm) was suspended in the solution for 12 h at room temperature. The surface of the Zn foil was washed 3 times with water and 3 times with ethanol. Subsequently, the Zn foil was dried with flowing N₂ and stored for future use.

### Example 3: Characterization of surface

The surfaces of the samples were characterized by SEM (JEOL JSM-7400E) and XRD (PANalytical X-ray diffractometer, X'pert PRO, with Cu Kα radiation at 1.5406 A). Prior to SEM, the samples were coated with thin Pt film using high resolution sputter coater (JEOL, JFC-1600 Auto Fine Coater). Coating conditions: For sample testing (20 mA, 30 s). For Pt coated sample for antibacterial testing (20 mA, 60 s).

Nano-patterns on copper substrate was prepared by treatment of copper foil in a (NH₄)₂S₂O₈ and NaOH solution at room temperature (see Example 1), 2 types of nanostructures were grown on copper substrate. As shown in Fig. 1, when copper foil was treated with lower concentration of the solution for 15 min, nanotubes array was grown. The nanotube array grew upwards and covered the whole area of the copper substrate compactly. Each tube was 5-7 µm in length with an open and sharp tip of -100-200 nm diameter. XRD confirmed the structure was Cu(OH)₂ with orthorhombic phase (JCPDS Card No. 13-0420). When the cupper foil was treated with higher concentration of the solution at ambient temperature, blade-like structure was formed on the Cu surface, with sharp edge standing upward. XRD confirmed the structure to be monoclinic symmetry of CuO on copper. (JCPDS Card No. 48-1548).

Similarly, a nano-patterned zinc surface was prepared by using a simple method (see Example 2). By treatment of a zinc foil in Zn(NO₃)₂ and KOH solution, ZnO nano-needles array was grown on the zinc substrate as shown below in Fig. 2. After treatment in the solution for 12 hours at room temperature, highly oriented uniform nano-needles array was formed on the surface. Further study showed that the needles were typically 1-2 µm in length. The diameters of the needle tips and roots are 10-40 nm and 100-200 nm, respectively. XRD analysis confirmed that the nano-needles are wurtzite ZnO structure. A strong diffraction peak at 34.4 ° (002) was present, indicating the highly preferential growth of ZnO nanoneedles along c-axis.

### Example 4: Bacterial growth conditions and sample preparation

*E. coli, S. aureus, and C. albicans* were obtained from American Type Culture Collection (ATCC-8739). Prior to each bacterial experiment, bacterial cultures were refreshed on nutrient agar from stock. Fresh bacterial suspensions were grown overnight at 37 °C in 5 ml of TSB (*E. coli and S. aureus*) or 5 ml YM broth for *C*. *albicans.* Bacterial cells were collected at the logarithmic stage of growth and the suspensions were adjusted to OD₆₀₀ = 0.07.

### Example 5: JIS killing efficacy testing

The tested bacteria were suspended in 5 mL of respective nutrient broth and adjusted to OD₆₀₀ = 0.07. In order to cover the surface, 150 µL of cell suspensions was placed on the surfaces. Experiments were carried out in triplicate at 37 °C. After incubation with the surfaces, the respective cell suspensions were washed and diluted, and each dilution spread on two nutrient agar plates. Resulting colonies were then counted using standard plate counts techniques, and the number of colony forming units per mL was calculated. The number of colony forming units was assumed to be equivalent to the number of viable cells in suspension.

The antibacterial properties against *E. coli* were evaluated for nano-patterned Cu surfaces by using JIS Z 2801:2000 (Japanese Industrial Standard) method. As shown in Fig. 3, all the bacteria were killed after 1 h incubation on Cu(OH)₂ nanotubes surface. For the CuO nano-blade surface, 94.5% of *E. coli* bacteria were killed after 1h incubation and all bacteria were killed after 3 hours. In relation to the control, Cu foil with a flat surface, only 28% of bacteria were killed after 1 h and there were still about 35% of *E*. *coli* surviving after 3 h.

From Fig. 3, it was observed that the *E. coli* killing efficacy was in the order of Cu(OH)₂ nanotubes > CuO nano-blades > Cu foil, which indicated that the sharper the surface, the better the killing efficacy. Considering that the chemical composition of the 3 surfaces are different (Cu(OH)₂, CuO, and Cu), to exclude the composition effect, three surfaces were coated with Pt and Cu, respectively, and the *E*. *coli* killing profiles were re-evaluated.

Fig. 4(A) demonstrates the killing efficacy against *E. coli* for the Pt coated samples. It was shown that Cu foil with Pt coating significantly changed the bacteria killing profile. Without Pt coating, flat Cu foil killed 65% of E. coli after 3 hours (Fig. 3). While after Pt coating, *E. coli* kept on growing instead after 3 hour incubation (Fig. 4). For Cu(OH)₂ nanotubes and CuO nano-blade surface, the killing profiles were almost unchanged after Pt coating as compared with the uncoated surfaces. All the bacteria were killed after 3 hour incubation, as shown in Fig. 4(A). To further confirm this result, three samples were also coated with Cu by vacuum vapour deposition method. SEM results did not show any obvious morphological change after Cu coating. After coating, all the three samples have the same chemical composition of Cu on the nano-patterned surfaces. As shown in Fig. 4(B), the killing profile of Cu-coated flat Cu foil was similar to the uncoated sample shown in Fig. 3. The killing efficacy of Cu(OH)₂ nanotubes surface and CuO nano-blades surface were maintained or even increased after coating with Cu. As can be seen from Fig. 4(B), all the bacteria were killed after 1 h incubation with copper coated nanotube and nano-blade surfaces. All these results indicated that the bacteria killing properties of these samples are mainly or entirely contributed by the surface nanostructures rather than chemical component.

The antibacterial activity against *E*. *coli* was also tested for zinc foil and ZnO nanoneedles. As shown in Fig. 5, all the bacteria were killed on ZnO nano-needles surface after 6 h incubation. As control, *E. coli* on flat Zn foil kept on growing, indicating the non-biocidal property of Zn foil. This result again demonstrated that the nano-structured zinc surface kills bacteria efficiently via physical interaction.

In addition to *E. coli,* which represents Gram-negative bacteria, Gram-positive bacteria were also tested. The antibacterial properties against S. *aureus* were also tested, as shown in Fig. 6.

As demonstrated in Fig. 6, the killing profile for S. *aureus* was similar to that of *E. coli.* The Cu(OH)₂ nano-tubes surface and CuO nano-blades surface killed nearly all the bacteria after 1 hour incubation, while for flat Cu foil, 23% of bacteria remained alive even after 3 hours incubation. For ZnO nano-needles surface, all the S. *aureus* were killed after 6 hours incubation, while 70% of S. *aureus* remained surviving on the flat Zn surface.

*C. albicans* as a sample of fungi was also tested. The killing profile for *C. albicans* was very different from those of *E. coli* and S. *aureus.* As shown in Fig. 7, all the tested surfaces could kill *C. albicans.* After 24 hours incubation, the remaining *C. albicans* were 2% (Cu), 4% (Cu(OH)₂), 0.7% (CuO), 1.3% (Zn) and 2.8% (ZnO). The nanostructured surfaces did not exhibit faster killing efficacy as compared with the flat surface. This might due to the robust cell wall of fungus as compared with other bacteria. As control, *C*. *albicans* on 6-well plate grow 25 times after 24 hours incubation, indicating the non-antibacterial of plate substrate (results not shown).

### Example 6: Bacterial killing efficacy under washing machine condition

To simulate the washing process, *E. coli* was suspended in 5 ml of water and adjusted to OD₆₀₀ = 0.07. The testing surfaces, mounted on 3.5 cm circular discs, were immersed in 5 ml of 1: 10 diluted bacterial suspension for incubation intervals and shaken at a speed of 300 r/min. The cell suspensions were then sampled (100 µl) at discrete time intervals, serially diluted 1:10, and each dilution spread on two nutrient agar plates. Resulting colonies were then counted, and the number of colony forming units per mL was calculated.

As an example of potential application of the nano-patterned Cu and Zn surfaces in washing machine, the bacterial killing activities of these nano-structured surfaces were tested under a simulated washing machine condition. *E. coli,* water and nanostructured surface were put in a bacterial culture plate, under shaking at 300 r/min. Bacteria in the solution was monitored by plate counting technique. The results show that for Cu(OH)₂ nanotubes and CuO nano-blades surfaces, all the bacteria in water are killed within 30 min. For ZnO nanoneedles surface, 82% of *E*. *coli* was killed after 1 h. In the control experiment, i.e. washing water without the nano-structured surface, the bacteria were still alive after 24 h. This experiment clearly demonstrated the possibility of making the inner surface of a washing machine having antibacterial surface/properties. The surface would kill bacteria during the washing session (30 - 60 min).

In summary, surfaces with Cu(OH)₂ nanotubes, CuO nano-blades and ZnO nano-needles have been prepared by simple solution treatment of respective copper or zinc foil at room temperature. All surfaces are bactericidal against *E*. *coli.* Application of these artificial surfaces are also demonstrated in washing machine condition in water, where *E*. *coli* bacteria are completely killed within 30 min by Cu(OH)₂ nanotubes and CuO nano-blades surfaces.

### Industrial Applicability

The nano-patterned surfaces of the present application may be useful in providing non-chemical anti-bacteria properties. Such anti-bacterial nano-patterned surfaces may be used as alternative surface materials for frequently-touched surfaces, e.g. doorknobs, handles and sanitary fittings, to provide an environment which discourages or inhibits bacteria proliferation such as in a hospital setting.

Advantageously, this would reduce the reliance on synthetic chemical disinfectants which may undesirably result in secondary contamination and may cause serious drug-resistant superbugs to develop. The disclosed patterned surfaces also lend possibility to the provision of domestic household appliances and equipment possessing such patterned metal surfaces. The anti-bacteria surface may also be used in a number of cleaning applications, e.g. to render the inner chamber surface of household or industrial scale washing machine anti-bacterial. This may advantageously reduce or completely eliminate the requirement of synthetic detergents which may be harmful to the human body. Also, the cleaning time may be reduced which results in higher cleaning efficiency of the washing machine.

It will be apparent that various other modifications and adaptations of the invention will be apparent to the person skilled in the art after reading the foregoing disclosure without departing from the spirit and scope of the invention and it is intended that all such modifications and adaptations come within the scope of the appended claims.

## Claims

1. A copper substrate having a plurality of, ordered, integrally-formed surface structures prepared by a method comprising the steps of:
contacting a surface of the copper substrate with a reagent solution, the reagent solution comprising an alkali and an oxidizing agent;
wherein the oxidizing agent is selected from the group consisting of persulfates, nitrates, halogen compounds, hypohalites and permanganates;
and wherein the concentration of the oxidizing agent in the reagent solution is at least about 0.3 M.

2. The substrate of claim 1, wherein the mole ratio of the oxidizing agent/alkali is from about 1:10 to 1:30.

3. The substrate of any one of claims 1-2, wherein the oxidizing agent is a persulfate or wherein the alkali is NaOH or KOH.

4. The substrate of any one of claims 1-3, wherein the contacting step is conducted for a duration sufficient to produce the plurality of surface features or for a duration of from about 25 minutes to about 35 minutes.

5. The substrate of any one of claims 1-4, wherein contacting step comprises dipping said substrate into said reagent solution.

6. The substrate of any one of claims 1-5, wherein the surface structure comprises a length of from about 200 nm to 5 µm, breadth of from about 100 nm to 500 nm, said surface structure having a thickness which tapers from the surface of the substrate towards the distal end of the blade, wherein the thickness of the blade at the distal end is about 10 nm to 30 nm.

7. The substrate of any one of claims 1-6, wherein the surface of the copper substrate is reactive with the oxidizing agent to form an insoluble salt.

8. The substrate of any one of claims 1-7, wherein the surface structure comprises a monoclinic crystal structure or wherein said monoclinic crystal structure comprises an oxide salt.

9. A method of killing or inhibiting the growth of a microorganism, said method comprising the steps of:
providing a copper substrate of any one of claims 1-8;
contacting said microorganism with said copper substrate.

10. The method of claim 9, wherein the microorganism is gram-negative or gram-positive bacteria or wherein the gram-negative bacteria is selected from the group consisting of Escherichia, Shigella, and Salmonella or wherein the gram-positive bacteria is selected from the group consisting of Staphylococcus, Enterococcus and Streptococcus.

11. Use of the copper substrate of any one of claims 1-8 for providing antibacterial properties to an ex-vivo environment.

12. The use of claim 11 for providing bacteriostatic or bactericidal purposes to said ex-vivo environment, is being a non-therapeutic use.

13. The use of any one of claims 11-12, wherein the antibacterial substrate is capable of killing or inhibiting the growth of gram-negative and gram-positive bacteria.

14. The use of claim 13, wherein the gram-negative bacteria is selected from the group consisting of Escherichia, Shigella, and Salmonella.

15. The use of claim 13, wherein the gram-positive bacteria is selected from the group consisting of Staphylococcus, Enterococcus and Streptococcus.
